# EUROPÄISCHE PATENTSCHRIFT

(11) **EP 0 559 662 B1**
(45) Veröffentlichungstag und Bekanntmachung des Hinweises auf die Patenterteilung: **20.07.1994**
(21) Anmeldenummer: 91919048.8
(22) Anmeldetag: 04.11.1991
(51) Int. Cl.: A61M 25/00

(54) **KATHETER**
CATHETER
CATHETER

(30) Priorität: 27.11.1990 DE 4037641
(43) Veröffentlichungstag der Anmeldung: 15.09.1993
(73) Patentinhaber: Haindl, Hans, Dr., D-30974 Wennigsen (DE)
(72) Erfinder: Haindl, Hans, Dr., D-30974 Wennigsen (DE)
(74) Vertreter: Leine, Sigurd, Dipl.-Ing.
(86) Internationale Anmeldenummer: EP9102071
(87) Internationale Veröffentlichungsnummer: WO9209326

(56) Entgegenhaltungen:
- EP-A- 0 386 408
- CA-A- 1 219 785
- US-A- 4 072 146
- US-A- 4 406 656
- US-A- 4 705 501

## Beschreibung

Die Erfindung betrifft einen Katheter der im Oberbegriff des Anspruchs 1 genannten Art.

Durch die Druckschrift "Medcomp" der Firma Medical Components Inc. 14999 Delp Drive Harleysville, PA 19438, USA, S. 4 ist ein Doppellumenkatheter bekannt, der aus einem querschnittssteifen Schlauch besteht, in dem "Seite an Seite" ein weiterer, querschnittssteifer, dünnerer Schlauch verläuft, der fest mit der Innenwandung des äußeren Schlauchs verbunden ist. Zwischen dem inneren und dem äußeren Schlauch ist ein Lumen gebildet, während das zweite Lumen innerhalb des inneren Schlauchs gebildet ist. Auf S. 6 der genannten Firmendruckschrift ist ein weiterer, doppellumiger Katheter gezeigt und beschrieben, der im Prinzip wie der zuerst beschriebene Katheter aufgebaut ist, bei dem jedoch der innere Schlauch lose und koaxial in dem äußeren Schlauch verläuft.

Durch die Patentschrift US 4 406 656 ist ein Katheter der betreffenden Art bekannt, der über seine Länge biegsam ist, in seinem inneren jedoch querschnittssteife Teile aufweist. An den Außenflächen dieser Querschnittsteile sind kollabierbare Querschnittsteile angebracht, die in sich jeweils ein Lumen zum Transport einer Flüssigkeit aufweisen. Diese kollabierbaren äußeren Wandungsteile beeinträchtigen die Handhabung und insbesondere die Einführung eines solchen Katheters beträchtlich. Darüber hinaus können die dünnen, kollabierbaren Wandungsteile leicht beschädigt werden. Bei zu hoher Druckbeanspruchung können sie platzen. Da die querschnittssteifen Bereiche sich im Inneren des Katheters befinden, ist die Biegsamkeit verhältnismäßig groß, wie das in der Praxis unerwünscht ist.

Durch die Druckschrift WO 8810128-A und durch die Druckschrift "CERTOFIX DUO/TRIO" der Firma B. Braun, Melsungen AG in D-3508 Melsungen sind Katheter der betreffenden Art bekannt, die aus einem Schlauch bestehen, der über seine Länge biegsam, im Querschnitt jedoch im wesentlichen steif ist und in dem drei unabhängige Lumina verlaufen.

In der Medizin besteht ein starker Bedarf nach mehrlumigen Kathetern, um inkompatible Medikamente durch einen einzigen Katheter getrennt voneinander einführen zu können und außerdem um eine parallele infusion, sowohl mit Schwerkraft als auch mit einer Pumpe, zu ermöglichen. Alle oben beschriebenen bekannten Katheter haben einen verhältnismäßig großen Durchmesser, da ihr Gesamtquerschnitt im wesentlichen der Summe der Einzelquerschnitte der einzelnen Lumina entspricht. Durch den größeren Querschnitt und durch die die einzelnen Lumina im Inneren des Katheters voneinander trennenden Stege ergibt sich außerdem aufgrund des erhöhten Widerstandsmoments eine höhere Steifigkeit. Weiterhin besteht der Nachteil, daß die durch die Stege zwischen den Lumina gebildete Versteifung das Knickverhalten des Katheters zumindest in einigen Biegerichtungen negativ beeinflußt. Aus diesem Grunde müssen die Wandstärken solcher mehrlumiger Katheter größer sein als bei Einlumenkathetern. Hinzu kommt, daß der Strömungswiderstand in den Lumina erhöht ist, da die Wandungsfläche im Verhältnis zu einem runden, einlumigen Katheter erhöht ist. Dies gilt insbesondere für die eingangs beschriebenen bekannten Katheter, bei denen das Lumen, das zwischen dem inneren und dem äußeren Schlauch gebildet ist, eine unverhältnismäßig große Umfangsfläche hat. Bekanntlich ist der Strömungswiderstand wesentlich durch die Größe der Umfangsfläche bestimmt.

Ein weiterer Nachteil der beschriebenen Mehrlumenkatheter besteht darin, daß, wie Statistiken zeigen, bei ihrer Verwendung das Risiko für den Patienten erhöht ist. Verantwortlich hierfür ist vor allem der große Durchmesser, der eine verhältnismäßig große Punktion erforderlich macht und in einem Gefäß dessen Querschnitt verengt. Außderdem werden durch die erhöhte Biegesteifigkeit in stärkerem Maße Biegekräfte auf die Punktionsöffnung und das Gefäß übertragen.

Der Erfindung liegt die Aufgabe zugrunde, einen Katheter der betreffenden Art mit wenigstens zwei Lumina zu schaffen, dessen Querschnitt im Verhältnis zu den bekannten mehrlumigen Kathetern verkleinert und dessen Biegesteifigkeit vermindert und bei dem außerdem das Risiko für den Patienten verringert ist.

Die der Erfindung zugrundeliegende Aufgabe wird durch die im Kennzeichen des Anspruchs 1 angegebene Lehre gelöst.

Die Erfindung beruht auf dem Grundgedanken, daß die einzelnen Lumina eines mehrlumigen Katheters nicht gleichzeitig mit ihrer vollen Durchflußkapazität benutzt werden oder werden müssen. Innerhalb eines im wesentlichen querschnittssteifen Umfangsbereichs, der wie bei einem einlumigen schlauchförmigen Katheter die gewünschte Handhabung gewährleistet, ist der Querschnitt der Einzellumina variabel und dies wird dadurch bewirkt, daß die Trennwand zwischen den beiden Lumina in der Querschnittsebene dehnbar und/oder verformbar ist. Dadurch ist es möglich, in einem Lumen durch gegenüber dem anderen Lumen bzw. den anderen Lumina erhöhten Druck dessen Querschnitt auf Kosten des anderen Lumens bzw. der anderen Lumina zu erhöhen. Entsprechendes gilt dann für das andere Lumen bzw. die anderen Lumina. Bei einem dreilumigen Katheter ergeben sich weitere Möglichkeiten, in dem beispielsweise zwei Lumina ihren Querschnitt auf Kosten des dritten Lumens erhöhen können oder umgekehrt. Bei Kathetern mit noch mehr Lumina ergeben sich zusätzliche Variationsmöglichkeiten.

Die Trennwände erhöhen das Widerstandsmoment des Gesamtkatheters aufgrund ihrer Dehnbarkeit und/oder Verformbarkeit nicht oder nur unwesentlich. Dadurch sind die Eigenschaften und die Handhabung gegenüber der einfachsten und günstigsten Form eines Katheters, nämlich eines Schlauches, praktisch unverändert. Entsprechend ist die Handhabung für den Arzt einfach und das Risiko für den Patienten gering.

Die innere Trennwand zwischen den einzelnen Lumina kann beliebige Ausführungsformen haben. Nach der Lehre des Anspruchs 2 bildet die Trennwand einen inneren Schlauch, der innerhalb eines äußeren Schlauches verläuft, der den im wesentlichen querschnittssteifen Umfangsbereich des Katheters bildet. Wird das Lumen zwischen dem inneren Schlauch und dem äußeren Schlauch durch eine Flüssigkeit mit Druck beaufschlagt, so faltet sich der innere, verformbare und flexible Schlauch teilweise oder ganz zusammen, so daß sich das Lumen zwischen dem inneren Schlauch und dem äußeren Schlauch praktisch über die gesamte lichte Weite des äußeren Schlauches erstreckt. Wird der innere Schlauch mit einer Flüssigkeit unter einem Druck beaufschlagt, der größer ist als der Druck zwischen dem inneren Schlauch und dem äußeren Schlauch, so dehnt sich der innere Schlauch auf seinen gesamten Querschnitt aus. Ist der innere Schlauch nicht nur flexibel, was bei einem dünnen, aber zugfesten Material der Fall ist, sondern dehnbar, so kann sich bei entsprechender Druckbeaufschlagung des inneren Schlauches dessen Querschnitt bis hin zu dem inneren Querschnitt des äußeren Schlauches erweiteren. In diesem Falle steht für beide variable Lumina im Extremfall der gesamte lichte Querschnitt des äußeren Schlauches zur Verfügung.

Bei der zuvor genannten Ausführungsform kann der innere Schlauch lose in dem äußeren Schlauch verlaufen. Der innere Schlauch kann aber auch teilweise mit dem äußeren Schlauch verbunden sein. In dem äußeren Schlauch können auch gleichzeitig mehrere innere Schläuche verlaufen.

Eine andere Ausführungsform des erfindungsgemäßen Grundgedankens ist im Anspruch 6 angegeben. Danach erstreckt sich die Trennwand diametral oder als Sekante in einem im wesentlichen runden, den querschnittssteifen Umfangsbereich bildenden Schlauch, und sie ist länger als der Durchmesser bzw. die Sekante. Dies gilt im wesentlichen für den Fall, daß der Steg weniger dehnbar als verformbar ist. Da er länger als der Durchmesser bzw. die Sekante ist, nimmt er, wenn kein Druck beaufschlagt ist, eine beliebige Form, z. B. eine Schlangen- oder Bogenform an. Bei Druck in einem Lumen (oder bei erhöhtem Druck gegenüber dem anderen Lumen) verformt sich der Steg im Sinne einer Vergrößerung des druckbeaufschlagten Lumens und nimmt in Abhängigkeit von seiner Länge im Extremfall eine Form an, in der er sich an die Innenwandung des äußeren Schlauches anlegt und somit das nicht mit Druck beaufschlagte Lumen weitgehend zum Verschwinden bringt.

Eine andere Ausführungsform ist in Anspruch 8 angegeben, wonach die Trennwand wenigstens drei Teiltrennwände aufweist, die sternförmig zusammenlaufen und so wenigstens drei Lumina bilden. Verlaufen dabei die Teiltrennwände gestreckt, so sollten sie aus einem dehnbaren Material bestehen. Zweckmäßig ist es jedoch, wenn sie ähnlich wie bei der zuvor genannten Ausführungsform länger sind als die direkte Verbindungslinie zwischen ihrem gemeinsamen, zentralen Verbindungspunkt und dem Verbindungspunkt am äußeren Umfang. Ohne Druckbeaufschlagung verlaufen sie alle z. B. bogenförmig, wobei der Bogen jeweils vom Lumen höherer Druckbeaufschlagung zum Lumen ohne oder geringer Druckbeaufschlagung auslenken kann.

Das distale Ende des Katheters kann grundsätzlich beliebig ausgebildet werden. Bei der Ausführungsform, bei der die Trennwand einen inneren Schlauch bildet, ist dieser am distalen Ende des Katheters zweckmäßigerweise dicht mit dem vorzugsweise verdünnten äußeren Ende des äußeren Schlauches verbunden, und in dem äußeren Schlauch befindet sich entfernt von dem distalen Ende eine Öffnung. Auf diese Weise sind die distalen Öffnungen der beiden Lumina voneinander entfernt, was insbesondere für inkompatible Medikamente zweckmäßig ist.

Bei den Ausführungsformen, bei denen die Trennwand diametral oder als Sekante verläuft oder bei denen die Trennwände im wesentlichen radial verlaufen, ist es zweckmäßig, daß die Lumina im distalen Ende des Katheters bis auf eines verschlossen sind, während das verschlossene Lumen bzw. die verschlossenen Lumina entfernt von dem distalen Ende des Katheters in vorzugsweise unterschiedlichen Entfernungen davon Durchflußöffnungen durch den äußeren, querschnittssteifen Umfangsbereich aufweisen. Auch bei mehreren Lumina ist also eine Trennung der distalen Mündungsöffnungen möglich, um so mehrere nicht kompatible Medikamente voneinander räumlich trennen zu können.

Zum Zwecke des Anschlusses des proximalen Endes des erfindungsgemäßen Katheters ist es zweckmäßig, wenn die Lumina in ihrem Durchmesser am proximalen Ende des Katheters vergrößert und in diesem Bereich mit einem Anschlußstück zum Anschluß der Lumina versehen sind. Auf diese Weise sind die Lumina durch das Anschlußstück in ihrem Durchmesser nicht beeinträchtigt. Die Vergrößerung der Durchmesser ist zweckmäßigerweise durch Intervallextrusion oder durch Aufweitung mittels eines beheizten Werkzeugs bewirkt.

Zum Anschluß eines erfindungsgemäßen Katheters, bei dem die Trennwand einen Schlauch in einem äußeren Schlauch bildet, ist es zweckmäßig, daß am proximalen Ende der innere Schlauch den äußeren Schlauch überragt. Beide Enden der Schläuche sind dicht in koaxiale Ausnehmungen eines Ansatzstückes eingesetzt, wobei sich jeweils hinter den Enden der Schläuche ein Raum befindet, der mit einem Anschluß verbindbar oder verbunden ist.

Anhand der Zeichnung soll die Erfindung an Ausführungsbeispielen näher erläutert werden.
**Fig. 1** zeigt eine erste Ausführungsform der Erfindung,
**Fig. 2** zeigt eine gegenüber der Ausführungsform nach Fig. 1 abgewandelte Ausführungsform,
**Fig. 3** zeigt im Längsschnitt die Ausbildung des distalen Endes des Katheters gemäß Fig. 1,
**Fig. 4** zeigt im Längsschnitt die Ausführung des distalen Endes des Katheters gemäß Fig. 2,
**Fig. 5** zeigt im Längsschnitt das proximale Ende des Katheters gemäß Fig. 1,
**Fig. 6** zeigt im Querschnitt eine dritte Ausführungsform des Katheters ohne Druckbeaufschlagung,
**Fig. 7** zeigt im Schnitt den Katheter gemäß Fig. 6 mit Druckbeaufschlagung in einem Lumen,
**Fig. 8** zeigt im Querschnitt eine vierte Ausführungsform der Erfindung ohne Druckbeaufschlagung,
**Fig. 9** zeigt im Querschnitt die Ausführungsform gemäß Fig. 8 mit Druckbeaufschlagung in einem Lumen,
**Fig. 10** zeigt im Längsschnitt die Ausbildung des proximalen Endes des Katheters gemäß Fig. 6,
**Fig. 11** ist ein Schnitt XI-XI durch Fig.10 und
**Fig. 12** ist ein Schnitt XII-XII durch Fig. 10.

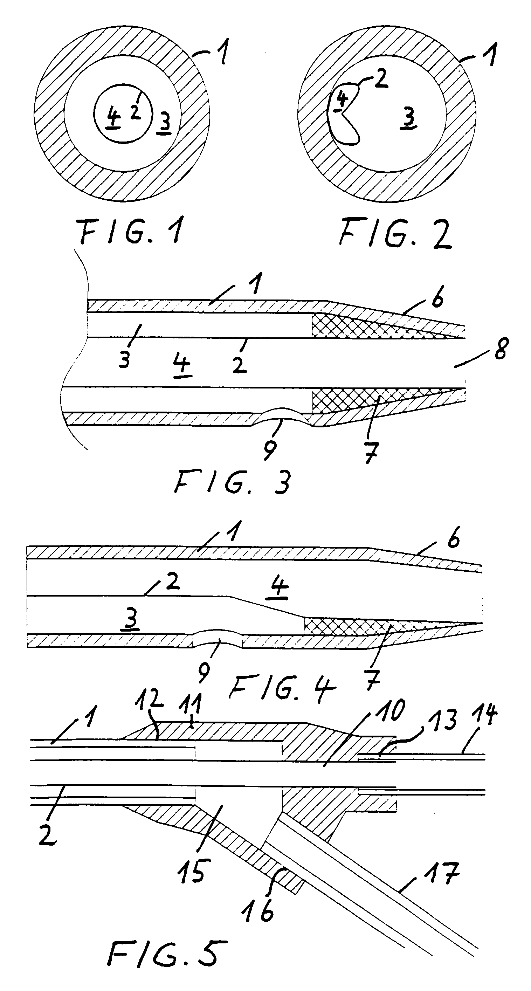

**Fig. 1** zeigt im Querschnitt einen Katheter, der aus einem äußeren Schlauch 1 und einem inneren Schlauch 2 besteht. Zwischen den Schläuchen 1 und 2 ist ein Lumen 3, und im Inneren des inneren Schlauches 2 ist ein Lumen 4 gebildet. Der äußere Schlauch hat eine verhältnismäßig große Wandstärke und ist wie herkömmliche Schlauchkatheter im wesentlichen querschnittssteif, über seine Längsausdehnung jedoch biegsam. Der innere Schlauch 2 hat eine sehr geringe Wandstärke und ist daher sehr leicht verformbar.

Wird bei Gebrauch eines Katheters gemäß Fig. 1 das Lumen 3 mit einer Flüssigkeit mit einem Druck beaufschlagt, während das Lumen 4 mit einem geringeren oder keinem Druck beaufschlagt ist, so faltet sich der dünne, innere Schlauch 2 zusammen. Er kann sich im Extremfall völlig zusammenfalten, so daß sich das Lumen 3 auf den gesamten lichten Querschnitt des äußeren Schlauches 1 ausdehnt. Wird danach das Lumen 4 mit Druck beaufschlagt, während das Lumen 3 mit einem kleineren oder keinem Druck beaufschlagt ist, so dehnt sich der innere Schlauch 2 wieder auf seinen vollem Umfang aus, so daß das Lumen 4 wieder seine volle Größe hat. Ist die Wandung des inneren Schlauches 2 dehnbar, so kann sich bei entsprechendem Druck in dem Lumen 4 dieses noch weiter auf Kosten des Lumens 3 vergrößern, es ist sogar möglich, daß sich der Schlauch 2 soweit ausdehnt, daß er sich über den gesamten Umfang an die Innenwandung des äußeren Schlauches 1 anlegt. Das Lumen 4 hat in diesem Fall den gleichen Querschnitt wie im vorgenannten Falle das Lumen 3. Die Lumina 3 und 4 können also wechselweise den gesamten lichten Querschnitt des äußeren Schlauches 1 einnehmen.

Fig. 2 zeigt im Querschnitt eine Ausführungsform ähnlich gemäß Fig. 1. Gleiche Teile sind mit gleichen Bezugsziffern versehen. Der Unterschied besteht darin, daß der Schlauch 2 über einen Teil seines Umfangs an einer Innenwandung 5 des äußeren Schlauches 1 befestigt ist. Er hat damit eine weitgehend definierte Lage innerhalb des äußeren Schlauches 1. In der in Fig. 2 dargestellten Lage ist die Wandung des inneren Schlauches 2 etwas nach innen gebogen. Dies kann die Ruhelage sein, sich aber auch dann ergeben, wenn das Lumen 3 mit Druck beaufschlagt ist.

Fig. 3 zeigt im Längsschnitt ein distales Ende des Katheters gemäß Fig. 1. Der äußere Schlauch 1 weist eine konische Verjüngung 6 bis auf einen Durchmesser entsprechend dem äußeren Durchmesser des inneren Schlauches 2 auf, in deren Bereich der innere Schlauch 2 durch Material 7, beispielsweise Klebstoff oder einen aushärtenden Kunststoff, mit dem äußeren Schlauch 1 verbunden ist. Entfernt von einer Mündung 8 des inneren Schlauches 2 befindet sich hinter dem Material 7 in dem äußeren Schlauch 1 eine Durchflußöffnung 9.

Fig. 4 zeigt ein distales Ende eines Katheters gemäß Fig. 2 ähnlich wie in Fig. 3. Gleiche oder sich entsprechende Teile sind mit gleichen Bezugsziffern versehen. Der Unterschied besteht darin, daß der innere Schlauch 2 nicht koaxial wie bei Fig. 3 verläuft, sondern an der Innenwandung 5 des äußeren Schlauches 1 befestigt ist.

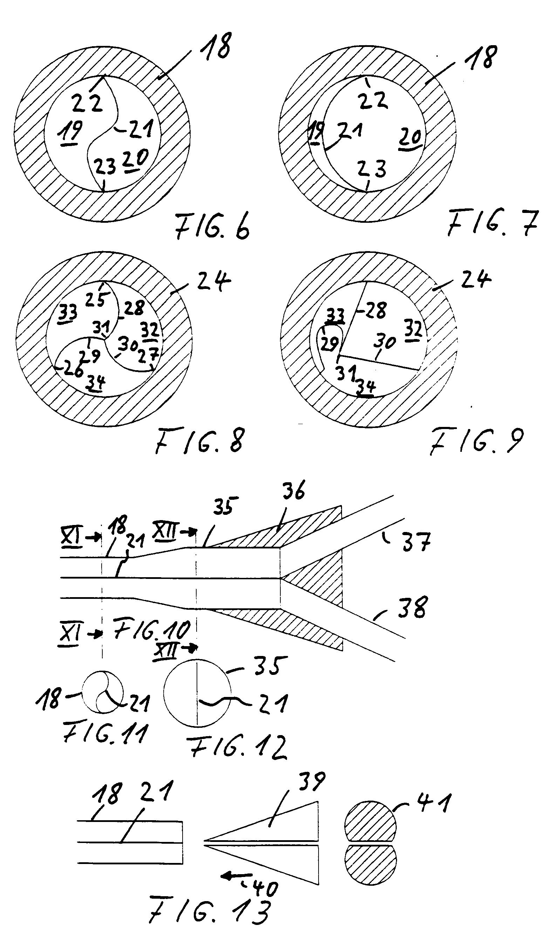

Fig. 5 zeigt im Längsschnitt die Ausbildung des proximalen Endes des Katheters gemäß Fig. 1. Der innere Schlauch 2 überragt den äußeren Schlauch 1 und mundet dicht in einer Ausnehmung 10 eines Anschlußstückes 11, in dem sich außerdem koaxial zu der Ausnehmung 10 eine weitere Ausnehmung 12 befindet, in der dicht der äußere Schlauch 1 mündet. Die Ausnehmung 10 weist eine Erweiterung 13 auf, in der ein Ende eines Anschlußschlauches 14 sitzt und dicht mit dem hinteren Ende des inneren Schlauches 2 verbunden ist.

Im Bereich zwischen den Ausnehmungen 10 und 12 befindet sich in dem Anschlußstück 11 ein Ringraum 15, der in eine Ausnehmung 16 mündet, in der dicht ein Anschlußschlauch 17 eingesetzt ist.

Fig. 6 zeigt im Schnitt eine Ausführungsform mit einem äußeren, querschnittssteifen Umfangsbereich 18, der im wesentlichen schlauchförmig ist und in dem sich zwei Lumina 19 und 20 befinden, die durch eine Trennwand 21 getrennt sind, die sich zwischen zwei diametral gegenüberliegenden Punkten 22 und 23 erstreckt. In der Darstellung in Fig. 6 sind die beiden Lumina 19 und 20 mit gleichem oder keinem Druck beaufschlagt. Der Steg 21 hat eine S-Form, ist also länger als der diametrale Abstand zwischen den Punkten 22 und 23. Die Trennwand 21 ist leicht verformbar. Bei Druckbeaufschlagung beispielsweise des Lumens 20 weicht die Trennwand 21 im Sinne einer Vergrößerung des Lumens 20 aus und nimmt eine Form ein, wie sie in Fig. 7 verdeutlicht ist. Es ist erkennbar, daß das Lumen 20 in seinem Querschnitt wesentlich gegenüber dem Lumen 19 vergrößert ist. Ist der Druck in dem Lumen 19 gegenüber dem in dem Lumen 20 größer, so liegen die Verhältnisse natürlich umgekehrt.

Fig. 8 zeigt im Schnitt ein Ausführungsbeispiel ähnlich gemäß Fig. 6. Ein Umfangsbereich 24 hat die Form eines querschnittssteifen Schlauches, in dem sich sternförmig oder radial ausgehend von Punkten 25, 26, 27 verformbare Trennwände 30 erstrecken, die in einem Punkt 31 dicht zusammenlaufen. Auf diese Weise sind Lumina 32, 33 und 34 gebildet.

In der in Fig. 8 gezeigten Lage befindet sich in den Lumina 32, 33 und 34 kein oder gleicher Druck. Die Trennwände 28, 29 und 30 verlaufen dabei bogenförmig, können aber auch eine andere beliebige Form in Abhängigkeit von ihrer Verformbarkeit einnehmen. In jedem Fall sind sie länger als der radiale Abstand zwischen dem Punkt 31 und jeweils den Punkten 25, 26 und 27.

Wird das Lumen 32 mit einem Druck beaufschlagt, während die Lumina 33 und 34 mit geringerem oder keinem Druck beaufschlagt sind, so strecken sich, wie das in Fig. 9 gezeigt ist, die Trennwände 28 und 30, während sich die Trennwand 29 zusammenfaltet. Dadurch vergrößert sich das Lumen 32, während sich die Lumina 33 und 34 verkleinern. Je nach der Größe des Drucks in dem Lumen 32 ist es auch möglich, daß sich die Trennwande 28 und 30 im Sinne einer Vergrößerung des Lumens 32, also von dem Lumen 32 weg, krümmmen, wie das durch gestrichelte Linien 28′ und 30′ angedeutet ist.

Ist der Druck in einem der anderen Lumina 32,34 größer, so ergeben sich entsprechende Verhältnisse.

Fig. 10 zeigt das proximale Ende des Katheters gemäß Fig. 6 im Längsschnitt, Fig. 11 ist ein Schnitt XI-XI durch Fig. 10 und Fig. 12 ist ein Schnitt XII-XII durch Fig. 10. Durch alle drei Figuren 10, 11 und 12 wird deutlich, daß der Querschnitt des Katheters im Bereich eines Endes 35 erweitert ist und dicht in einem Anschlußstück 36 sitzt, das die Verbindung zu zwei Anschlußschläuchen 37 und 38 herstellt. Das distale Ende des Katheters kann genauso ausgebildet sein, wie das in Fig. 4 gezeigt ist.

Fig. 13 verdeutlicht die Herstellung des erweiterten Endes 35 durch Einführen eines keilförmigen, warmen Werkzeugs 39 in Richtung eines Pfeiles 40. Ganz rechts ist in Fig. 13 ein Querschnitt 41 des Werkzeugs 39 gezeigt.

## Patentansprüche

1. Katheter, der teilweise querschnittssteif ist und der wenigstens zwei Lumina aufweist, die durch eine Trennwand voneinander getrennt sind, **dadurch** **gekennzeichnet**, daß der Katheter in seinem Umfangsbereich (1, 18, 24) querschnittssteif ist, daß die Trennwand (2, 21, 28-30) in der Querschnittsebene dehnbar und/oder verformbar ist und daß die wenigstens zwei Lumina (3, 4; 19, 20; 32-34) jeweils beidseitig offen ausgebildet sind.

2. Katheter nach Anspruch 1, **dadurch gekennzeichnet**, daß die Trennwand einen inneren Schlauch (2) bildet, der innerhalb eines äußeren Schlauches (1) verläuft, der den im wesentlichen querschnittssteifen Umfangsbereich des Katheters bildet.

3. Katheter nach Anspruch 2, **dadurch gekennzeichnet**, daß der innere Schlauch (2) lose in dem äußeren Schlauch (1) verläuft.

4. Katheter nach Anspruch 2, **dadurch gekennzeichnet**, daß der innere Schlauch (2) wenigstens über einen Teil seines Umfangs mit dem äußeren Schlauch (1) verbunden ist.

5. Katheter nach Anspruch 2, **dadurch gekennzeichnet**, daß mehrere innere Schläuche in dem äußeren Schlauch (1) angeordnet sind.

6. Katheter nach Anspruch 1, **dadurch gekennzeichnet**, daß die Trennwand (21) sich diametral oder als Sekante in einem im wesentlichen runden, den querschnittssteifen Umfangsbereich (18) bildenden Schlauch erstreckt und länger als der Querschnitt bzw. die Sekante ist.

7. Katheter nach Anspruch 6, **dadurch gekennzeichnet**, daß die Trennwand (21) so lang ist, daß sie sich zur einen oder anderen Seite bis an eine benachbarte Innenwand eines Lumens anlegen kann.

8. Katheter nach Anspruch 1, **dadurch gekennzeichnet**, daß die Trennwand (21) wenigstens drei Teiltrennwände (28, 29, 30) aufweist, die sternförmig aufeinander zulaufen und so wenigstens drei Lumina bilden.

9. Katheter nach Anspruch 3 oder 4, **dadurch gekennzeichnet**, daß der innere Schlauch (2) am distalen Ende des Katheters dicht mit einem vorzugsweise eine Verjüngung (6) aufweisenden äußeren Ende des äußeren Schlauches (1) verbunden ist und daß sich in dem äußeren Schlauch (1) entfernt von dem distalen Ende eine Durchflußöffnung (9) befindet.

10. Katheter nach Anspruch 6 oder 8, **dadurch gekennzeichnet**, daß die Lumina am distalen Ende des Katheters bis auf eines verschlossen sind, und daß das verschlossene Lumen bzw. die verschlossenen Lumina entfernt von dem distalen Ende des Katheters in vorzugsweise unterschiedlichen Entfernungen davon Durchflußöffnungen durch den äußeren Umfangsbereich aufweist bzw. aufweisen.

11. Katheter nach Anspruch 1, **dadurch gekennzeichnet**, daß die Lumina (19,20) am proximalen Ende des Katheters vergrößert und in diesem Bereich mit einem Anschlußstück (36) zum Anschluß der Lumina (19, 20) versehen sind.

12. Katheter nach Anspruch 11, **dadurch gekennzeichnet**, daß die Vergrößerung der Querschnitte durch Intervallextrusion oder durch Aufweitung mittels eines Werkzeuges (39) bewirkt ist.

13. Katheter nach Anspruch 2, **dadurch gekennzeichnet**, daß am proximalen Ende des Katheters der innere Schlauch (2) den äußeren Schlauch (1) überragt, daß beide Enden der Schläuche (1, 2) dicht in koaxialen Ausnehmungen (10, 12) eines Anschlußstückes (11) eingesetzt sind, wobei sich jeweils hinter den Enden der Schläuche (1, 2) ein Ringraum (13, 15) befindet, der mit einem Anschluß oder einem Anschlußschlauch (14, 17) verbindbar oder verbunden ist.

## Claims

1. Catheter which is partly rigid in cross-section and comprises at least two lumens separated from each other by a partition, characterised in that the catheter is in its peripheral zone (1, 18, 24) rigid in cross-section, that the partition (2, 21, 28-39) is in the cross-sectional plane yieldable and/or deformable, and that the, at least two, lumens (3, 4; 19, 20; 32-34) are open at both ends.

2. Catheter according to claim 1, characterised in that the partition forms an inner tube (2) which extends inside an outer tube (1) which forms the peripheral zone of the catheter, the peripheral zone being substantially rigid in cross-section.

3. Catheter according to claim 2, characterised in that the inner tube (2) extends loosely inside the outer tube (2).

4. Catheter according to claim 2, characterised in that the inner tube (2) is along a portion of its periphery connected to the outer tube (1).

5. Catheter according to claim 2, characterised in that a plurality of inner tubes is situated inside the outer tube (1).

6. Catheter according to claim 1, characterised in that the partition (21) extends diametrally or as a secant in a tube, which is substantially round and forms the peripheral zone (18) rigid in cross-section, the partition being longer than the cross-section or the secant.

7. Catheter according to claim 6, characterised in that the partition (21) is so long, that it may lean at one or the other side up to against an adjacent inner wall of a lumen.

8. Catheter according to claim 1, characterised in that the partition (21) comprises at least three partial partitions (28, 29, 30), which extend towards each other in a star-shaped configuration and form thereby at least three lumens.

9. Catheter according to claim 3 or 4, characterised in that the inner tube (2) is at the distal end of the catheter connected in a leak-proof manner to an outer end of the outer tube (1), the outer end containing preferably a constriction (6), and that a through-flow opening (9) is provided in the outer tube (1) at a point spaced from the distal end.

10. Catheter according to claim 6 or 8, characterised in that, except for one, the lumens are closed at the distal end of the catheter, and that the closed lumen or lumens has or have through-flow openings, which extend through the peripheral zone and are spaced, preferably at different distances, from the distal end of the catheter.

11. Catheter according to claim 1, characterised in that the lumens (19, 20) are increased at the proximal end of the catheter and are provided at this region with a connector (36) for the connection of the lumens (19, 20).

12. Catheter according to claim 11, characterised in that the increase of the cross-sections is obtained by interval extrusion or by widening with a tool (39).

13. Catheter according to claim 2, characterised in that the inner tube (2) extends at the proximal end of the catheter beyond the outer tube (1), and that both ends of the tubes (1, 2) are inserted in a leak-proof manner into co-axial recesses (10, 12) in a connector (11), a ring-shaped space (13, 15) being provided behind the ends of the tubes (1, 2) which may be or is connected to a connector or a connection tube (14, 17).

## Revendications

1. Cathéter qui est partiellement rigide en section transversale et qui présente au moins deux passages qui sont séparés par une paroi de séparation,
caractérisé en ce que le cathéter est rigide en section transversale dans sa zone périphérique (1,18,24), que la paroi de séparation (2,21,28-30) est élastique et/ou déformable dans le plan de section transversale et que les au moins deux passages (3,4 ; 19,20 ; 32-34) sont réalisés respectivement ouverts des deux côtés.

2. Cathéter selon la revendication 1,
caractérisé en ce que la paroi de séparation forme un tuyau intérieur (2) qui s'étend à l'intérieur d'un tuyau extérieur (1) qui forme la zone périphérique, essentiellement rigide en section transversale, du cathéter.

3. Cathéter selon la revendication 2,
caractérisé en ce que le tuyau intérieur (2) s'étend de manière lâche dans le tuyau extérieur (1).

4. Cathéter selon la revendication 2,
caractérisé en ce que le tuyau intérieur (2) est relié, sur au moins une partie de sa périphérie, au tuyau extérieur (1).

5. Cathéter selon la revendication 2,
caractérisé en ce que plusieurs tuyaux intérieurs sont disposés dans le tuyau extérieur (1).

6. Cathéter selon la revendication 1,
caractérisé en ce que la paroi de séparation (21) s'étend diamétralement ou en forme de sécante dans un tuyau essentiellement rond formant la zone périphérique (18) rigide en section transversale et est plus long que la section transversale ou respectivement la sécante.

7. Cathéter selon la revendication 6,
caractérisé en ce que la paroi de séparation (21) a une longueur telle qu'elle peut se mettre d'un côté ou de l'autre sur une paroi intérieure adjacente d'un passage.

8. Cathéter selon la revendication 1,
caractérisé en ce que la paroi de séparation (21) présente au moins trois parois de séparation partielles (28,29,30) qui s'étendent l'une vers l'autre en forme d'étoile et forment ainsi au moins trois passages.

9. Cathéter selon la revendication 3 ou 4,
caractérisé en ce que le tuyau intérieur (2) est relié à l'extrémité distale du cathéter, de manière étanche, à une extrémité extérieure, présentant de préférence un rétrécissement (6), du tuyau extérieur (1) et qu'une ouverture de circulation (9) se trouve dans le tuyau extérieur (1), éloignée de l'extrémité distale.

10. Cathéter selon la revendication 6 ou 8,
caractérisé en ce que les passages, à l'exception d'un, sont fermés à l'extrémité distale du cathéter et que le passage fermé ou respectivement les passages fermés présente ou respectivement présentent des ouvertures de circulation à travers la zone périphérique extérieure, écartées de l'extrémité distale du cathéter à des écartements par rapport à elle de préférence différents.

11. Cathéter selon la revendication 1,
caractérisé en ce que les passages (19,20) sont agrandis à l'extrémité proximale du cathéter et sont munis dans cette zone d'une pièce de raccordement (36) pour le raccordement du passage (19,20).

12. Cathéter selon la revendication 11,
caractérisé en ce que l'agrandissement des sections transversales est effectué par extrusion fractionnée ou par élargissement au moyen d'un outil (39).

13. Cathéter selon la revendication 2,
caractérisé en ce que, à l'extrémité proximale du cathéter, le tuyau intérieur (2) dépasse le tuyau extérieur (1), que les deux extrémités des tuyaux (1,2) sont logés de manière étanche dans des évidements coaxiaux (10,12) d'une pièce de raccordement (11), un espace annulaire (13,15) se trouvant respectivement derrière les extrémités des tuyaux (1,2), qui peut être relié ou est relié à un raccordement ou à un tuyau de raccordement (14,17).
